# EUROPEAN PATENT SPECIFICATION

(11) **EP 0 301 107 B1**
(45) Date of publication and mention of the grant of the patent: **17.08.1994**
(21) Application number: 88901636.6
(22) Date of filing: 12.02.1988
(51) Int. Cl.: C12P 41/00

(54) **PROCESS FOR PREPARING D-ALANINE**
VERFAHREN ZUR HERSTELLUNG VON D-ALANIN
PROCEDE DE PREPARATION DE D-ALANINE

(30) Priority: 13.02.1987 JP 29709/87
(43) Date of publication of application: 01.02.1989
(73) Proprietor: TORAY INDUSTRIES, INC., Tokyo 103 (JP)
(72) Inventor: ITO, Noriko 1-302, Nippon Steel Kaminawa Shataku, Aichi 476 (JP); IMAMURA, Shinzo 2-1, Toray Matsuzono Shataku, Aichi 467 (JP); SATO, Haruyo, Aichi 454 (JP)
(74) Representative: Paget, Hugh Charles Edward
(86) International application number: JP8800139
(87) International publication number: WO8806188

(56) References cited:
- US-A- 3 871 959
- CHEMICAL ABSTRACTS, vol. 67, 1967, page 3980, abstract no. 42581d, Columbus,Ohio, US; K. OSHIMA et al.: "Preparation of optically active alanine from DL-alanine by yeast", & HAKKO TO TAISHA NO. 15, 89-94,DISCUSSION 94(1967)

## Description

The present invention relates to a method for producing industrially D-alanine by fermentation.

A method for producing D-alanine by cultivating a yeast in a medium containing both glucose and DL-alanine is disclosed by Oshima et al in "Amino Acid and Nucleic Acid", 15, 89-94 (1967). For such a yeast, an alkaline pH is recommended.

JP-B-20683/85 describes a method for producing about equal amounts of D-alanine and pyruvic acid in parallel by using a microorganism capable of oxidizing L-alanine in the presence of both glucose and DL-alanine. Again, a pH of 6.8-7 is employed.

Both conventional methods are excellent as methods for obtaining expensive D-alanine with high purity from inexpensive DL-alanine.

However, in Oshima et al supra, it was clearly stated that if the concentration of DL-alanine was 20 g/l or more, then not only was the growth of the strains obstructed, but its ability to selectively decompose was also decreased. Thus, because the concentration of the raw material DL-alanine is limited to an amount below 20 g/l, the yield of D-alanine can at most only be 10 g/l even if a theoretically quantitative amount can be achieved and therefore it is not an industrially favorable method.

In the method of JP-B-20683/85, the concentration of DL-alanine was at most 50 g/l and the yield of D-alanine was also low, namely, at most 17 g/l. The time required for cultivating the strains and oxidising the L-alanine was long, namely, 72 hours in all, so this method is also not industrially suitable. Moreover, in this method, it is necessary also to separate and remove a large amount of pyruvic acid in order to obtain D-alanine. This also renders it industrially unfavorable.

We have made extensive studies in an attempt to solve the above described problems and to offer an industrially favorable method for producing D-alanine, and have found that D-alanine in large amounts can be obtained by cultivating a specific yeast in a culture medium containing a specific carbon and nitrogen source.

A purpose of the present invention is to offer a method for producing D-alanine with high yield, namely in an almost theoretically quantitative amount, from DL-alanine, by a selective assimilation method using a microorganism.

Another purpose of the present invention is to offer a method for producing D-alanine with high yield by supplying a high concentration of DL-alanine to a culture medium without obstructing the growth of strains and without decreasing its ability to selectively decompose.

Another purpose of the present invention is to offer a method in which very little organic byproduct and organic impurity exists in the culture medium when the cultivation is completed.

Another purpose of the present invention is to offer an efficient method in which the time required for cultivating and assimilating a half of the DL-alanine can be shortened.

These and other purposes of the present invention will be clarified by the following detailed explanation.

These purposes can be achieved by a method for producing D-alanine by cultivating a yeast which belongs to the genus Candida, Saccharomycopsis, Pichia, Torulopsis, Cryptococcus, Hansenula or Trichosporon and has an ability to assimilate L-alanine substantially without assimilating D-alanine in a medium having a pH of 6.5 or less, containing, DL-alanine, used in an amount of from 60 to 200 g/l, as a combined source of both carbon and nitrogen and containing from 0 to less than 10 g/l of an additional carbon and/or nitrogen source, and by obtaining D-alanine from the cultivated substance.

Embodiments of the present invention will now be described in more detail.

As the yeast, those belonging to the genus Candida, Saccharomycopsis, Pichia, Torulopsis, Cryptococcus, Hansenula or Trichosporon can be used. Among these yeasts, such yeasts that can grow in a medium containing substantially DL-alanine as a combined single carbon and nitrogen source and at the same time have an ability to assimilate L-alanine and not to assimilate substantially D-alanine can be used in the present invention.

In the present invention, yeasts which do not substantially assimilate D-alanine include such yeasts that do assimilate only a small amount of D-alanine, to the extent that the effect of the present invention is not substantially lost, or that assimilate D-alanine without existence of L-alanine, after L-alanine has been completely assimilated.

For example, Candida humicola ATCC 36992, Candida rugosa ATCC 10571, Saccharomycopsis lipolytica ATCC 20306, Saccharomycopsis lipolytica IFO 0717, Cryptococcus laurentii ATCC 36832, Torulopsis candida ATCC 20284, Torulopsis glabrata IFO 0005, Pichia burtonii ATCC 20279, Pichia pastoris IFO 0948, Hansenula polymorpha ATCC 26012, Hansenula capsulata ATCC 16753, Trichosporon beigelii ATCC 36993 are employed.

The above ATCC numbers are the registration numbers of deposits at the American Type Culture Collection, 12301 Parklawn Drive, Rockville, Maryland 20852, USA and the IFO numbers are the registration numbers of deposits at the Institute of Fermentation, 17-35, Jusanbon-machi 2-chome, Yodogawa-ku, Osaki-shi, Osaka 532, Japan.

In a method the present invention, the cultivation is carried out in a medium containing substantially DL-alanine as a combined carbon and nitrogen source. Thus, in the method, as a carbon source and a nitrogen source in a medium, DL-alanine is used, but small amounts of other carbon sources (such as glucose) and/or nitrogen sources may be included to the extent that the effect of the present invention is not lost, namely, in an amount less than 10 g/l.

It is preferable that the amount of glucose included is as small as possible because the rate of assimilation of L-alanine becomes low if glucose at 10 g/l or more coexists in the culture medium as a carbon source.

In the culture medium, salts of metal ion can be added according to the microorganism to be used. As the metal ion, for example, Na⁺, K⁺, Ca²⁺, Mg²⁺, Fe²⁺, Ni²⁺, Zn²⁺, Co²⁺, Mn²⁺ and so on are cited and the salts of various inorganic acids such as sulfuric acid, hydrochloric acid, phosphoric acid and so on of these metal ions can be used.

The concentration of DL-alanine in a medium is 60 to 200 g/l. When the concentration of DL-alanine is low, the productive efficiency becomes worse; on the other hand when the concentration is high, the cultivation time becomes longer and the growing of microorganisms may be arrested.

The total amount of DL-alanine can be fed in the culture medium from the beginning, but because, if the concentration becomes high, the growing of microorganisms becomes low and the cultivation time therefore becomes long, a flow-adding cultivation in which the initial concentration is made to be 20 to 50 g/l and the remaining DL-alanine is dividedly fed is preferable.

The cultivation is carried out on the acidic side; ie at pH 6.5 or less. In the culture medium pH is usually adjusted to 5 when the cultivation starts, but the pH increases as the cultivation proceeds. Because the rate of recovery of D-alanine decreases if the cultivation is left as it is, it is necessary to adjust the pH to the acidic side.

The reason why the rate of recovery of D-alanine decreases if the pH becomes alkaline is considered to be that D-alanine is assimilated because alanine racemase or D-alanine-aminotransferase is activated.

Based on these reasons, the pH during cultivation is usually adjusted to 4 to 6.5, preferably 4.5 to 6.0. As the acid for the adjustment, for example, an aqueous solution of an inorganic acid such as phosphoric acid, sulfuric acid or hydrochloric acid is preferable.

In a published abstract of Oshima et al, supra, the conclusion was drawn that selective decomposition occurred on the high pH side during cultivation and selectivity rather became worse on the low pH side. For example, in Figure 4 in the above described entire article an example was shown in which selective decomposition by Torulopsis famata was completed at pH 8.5.

In the case where pH during cultivation is 6.5 or less, as in the method of the present invention, because ordinary bacteria hardly grow, there is an advantage in that contamination with saprophyte scarcely occurs during cultivation.

The cultivation temperature is usually 20 to 40°C, preferably 25 to 35°C. The cultivation is carried out with stirring and aeration. The amount of aeration is usually 0.5 to 2.0 vvm, preferably 0.6 to 1.2 vvm. If the amount of aeration is too small, there is a tendency that the assimilating speed of L-alanine becomes slow. If it is too large, the effect does not change, but it is then not desirable for the concentration of the culture medium to be higher and moreover vigorous foaming occurs because vaporization of the culture medium is rather accelerated.

The cultivation is usually finished when L-alanine is wholly assimilated. Whole assimilation of L-alanine can be detected by monitoring the amount of dissolved oxygen or analysing the amount of D-isomer and L-isomer of alanine. An acid is added to neutralize ammonia produced by assimilation of L-alanine during cultivation, but the addition of the acid becomes unnecessary after assimilation of L-alanine is completed. Therefore it can also be detected by monitoring the amount of acid added.

Because D-alanine is also gradually assimilated after L-alanine has been wholly assimilated, in some cases it is desirable that the end point of the cultivation is clearly detected.

After removing cells from the culture broth thus obtained by means of a centrifugal separation method, D-alanine is separated by a usual method.

For example, alanine is adsorbed with an ion-exchange resin "SK-1B"(manufactured by Mitsubishi Chemical Industries Co., Ltd.), by passing the medium through the resin, and thereafter well washed. Then, alanine is eluted with an aqueous ammonia solution and the eluate is concentrated. Refined D-alanine can be obtained by recrystallizing the crude D-alanine thus obtained with water.

The present invention exhibits the following effects.
(1) D-alanine can be obtained in high yield, namely with an almost theoretically quantitative amount from DL-alanine.
(2) Moreover, D-alanine can be obtained in high yield by supplying high concentration of DL-alanine to a culture broth without obstructing the growth of strains and without decreasing the ability of selective decomposition.
(3) Moreover, L-alanine having been consumed is mostly converted into carbon dioxide and water and neither organic by-product nor organic impurity exists substantially in the culture broth. It is therefore easy to separate and refine D-alanine.
(4) D-alanine can be efficiently obtained, as the time required for cultivating and assimilating a half of DL-alanine can be shortened.
(5) Because the cultivation is carried out under an acidic condition of pH 6.5 or less, contamination with saprophyte hardly occurs during cultivation.

The present invention will hereinafter be specifically explained by Examples.

In the Examples, DL analysis of alanine is carried out by High Performance Liquid Chromatography (hereinafter referred to as "HPLC"). The sample is prepared by esterifying alanine in powder with methanol-hydrochloric acid and reacting with 3,5-dinitrophenylisocyanate. The analysing condition of HPLC is an follows;

| | |
|---|---|
| Column | OA-1000(Sumitomo Chemical Co., Ltd.) |
| Mobile phase | n-Hexane :Dichloromethane : Ethanol (20:8:1) |
| Flow rate | 1ml/min |
| Detector | UV 254 nm |

### Example 1

50 ml of a medium containing 30 g/ℓ of dried bouillon (pH 6.0) were dividedly poured into an 1ℓ Erlenmeyer flask and sterilized at 120°C for 20 minutes to make a medium for cultivating species. One platinum loop of Candida humicola ATCC 36992 was inoculated on the medium and was cultivated with shaking at 30°C for a day. On the other hand, 1ℓ of a medium (pH 5.0) containing 100 g/ℓ of DL-alanine, 2 g/ℓ of potassium hydrogenphosphate, 0.5 g/ℓ of magnesium sulfate and 0.5 g/ℓ of powder yeast extract was put into a 3ℓ mini jar fermenter and sterilized to make a main culture medium. The above described culture broth was inoculated on it and was cultivated with stirring and aeration of 1.0 vvm at 30°C. During the cultivation, the pH was adjusted at 5.0±0.1 with 2N sulfuric acid. L-alanine was wholly assimilated for about 70 hours and 1.2 ℓiter of the culture broth containing 48g of D-alanine, 35g of ammonium sulfate was obtained.

After removing cells from this culture broth by means of a centrifugal separator at 10,000 rpm for 10 minutes, D-alanine was absorbed with an ion-exchange resin SK-1B (H type) by passing the medium through a column packed with the resin. After thoroughly washing the column with water, D-alanine was eluted with 4% aqueous ammonia solution. This eluate was concentrated under reduced pressure to obtain 45g of D-alanine. The optical purity was 99.6% ee or more on analysis by means of HPLC. The chemical purity was 99.4%.

### Example 2

In this case, among the procedures shown in Example 1, the concentration of DL-alanine in the main culture medium was changed to 80 g/ℓ and the others were the same as those of Example 1. The cultivation was finished for about 35 hours and about 1.2ℓ of the culture broth containing 38g of D-alanine and 28g of ammonium sulfate were obtained.

After removing cells from this culture broth by means of a centrifugal separator, a salt exchanging operation was carried out by adding and stirring 18.3g of calcium hydroxide for about 2 hours. This suspension was concentrated under reduced pressure to one third of the original amount and thereafter filtered to remove inorganic salts. A slight amount of metallic ion was absorbed on an ion-exchange resin SK-1B (ammonium type) by passing the filtrate through a column packed with the resin. Combining the eluate and the washing liquid of the column, the combined solution was concentrated to crystallize D-alanine out. 32g of refined D-alanine were thus obtained.

The optical purity and the chemical purity were 99.9% ee and 99.9% respectively.

### Comparison Example

A cultivation was carried out under the same conditions as the procedures shown in Example 2 except that 10 g/ℓ of glucose was added to the main culture medium. The cultivation was finished after about 54 hours and about 1.2ℓ of the culture medium containing 32g of D-alanine and 29g of ammonium sulfate. The solid obtained by treating 50ml of this culture medium with the same procedures as those of Example 5 to 14 was analyzed by means of HPLC and the optical purity of D-alanine thus obtained was found to be 99. 6% ee or more.

Industrially, D-alanine is useful for a medical raw material or a raw material of the sweetener "Alithame".

## Claims

1. A method for producing D-alanine by cultivating a yeast in a culture broth and obtaining the D-alanine from the culture broth characterised in that the yeast belongs to the genus Candida, Saccharomycopsis, Pichia, Torulopsis, Cryptococcus, Hansenula or Trichosporon and has an ability to assimilate L-alanine and not to assimilate D-alanine, and the culture broth has a pH of 6.5 or less, contains DL-alanine, used in an amount of from 60 to 200 g/l, as a combined source of both carbon and nitrogen, and contains from 0 to below 10 g/l of an additional carbon and/or nitrogen source.

2. A method according to Claim 1, wherein the yeast is of the species Candida humicola or Candida rugosa.

3. A method according to Claim 1 or Claim 2, wherein the cultivation is carried out at pH 4 to 6.5.

## Patentansprüche

1. Verfahren zur Herstellung von D-Alanin durch Züchten einer Hefe in einer Kulturbrühe und Gewinnen des D-Alanins aus der Kulturbrühe, dadurch gekennzeichnet, daß die Hefe den Gattungen Candida, Saccharomycopsis, Pichia, Torulopsis, Cryptococcus, Hansenula oder Trichosporon angehört und die Fähigkeit besitzt, L-Alanin zu assimilieren und D-Alanin nicht zu assimilieren, und die Kulturbrühe einen pH-Wert von 6,5 oder weniger aufweist, als kombinierte Quelle sowohl von Kohlenstoff als auch von Stickstoff in einer Menge von 60 bis 200 g/l verwendetes DL-Alanin enthält, und von 0 bis unter 10 g/l einer zusätzlichen Kohlenstoff- und/oder Stickstoffquelle enthält.

2. Verfahren nach Anspruch 1, worin die Hefe der Spezies Candida humicola oder Candida rugosa angehört.

3. Verfahren nach Anspruch 1 oder Anspruch 2, worin das Züchten bei einem pH-Wert von 4 bis 6,5 ausgeführt wird.

## Revendications

1. Un procédé pour produire de la D-alanine en cultivant une levure dans un bouillon de culture et en obtenant la D-alanine à partir du bouillon de culture, caractérisé en ce que la levure appartient au genre Candida, Saccharomycopsis, Pichia, Torulopsis, Cryptococcus, Hansenula ou Trichosporon et a une aptitude à assimiler la L-alanine et à ne pas assimiler la D-alanine, et le bouillon de culture a un pH de 6,5 ou moins, contient de la DL-alanine utilisée en une quantité de 60 à 200 g/l comme source combinée à la fois de carbone et d'azote et contient de 0 à moins de 10 g/l d'une source supplémentaire de carbone et/ou d'azote.

2. Un procédé selon la revendication 1, dans lequel la levure est de l'espèce Candida humicola ou Candida rugosa.

3. Un procédé selon la revendication 1 ou 2, dans lequel la culture est exécutée à un pH de 4 à 6,5.
